(19) Europäisches Patentamt
European Patent Office
Office européen des brevets

(11) Veröffentlichungsnummer: **0 059 894**
**B1**

(12) # EUROPÄISCHE PATENTSCHRIFT

(45) Veröffentlichungstag der Patentschrift: **19.09.84**

(51) Int. Cl.³: **C 07 D 249/08,** A 01 N 43/50

(21) Anmeldenummer: **82101460.2**

(22) Anmeldetag: **26.02.82**

(54) **Triazolylalkyl-thioether, Verfahren zu ihrer Herstellung sowie ihre Verwendung als Pflanzenwachstumsregulatoren und Fungizide.**

(30) Priorität: **07.03.81 DE 3108770**

(43) Veröffentlichungstag der Anmeldung:
**15.09.82 Patentblatt 82/37**

(45) Bekanntmachung des Hinweises auf die Patenterteilung:
**19.09.84 Patentblatt 84/38**

(84) Benannte Vertragsstaaten:
**AT BE CH DE FR GB IT LI NL**

(56) Entgegenhaltungen:
**EP - A - 0 000 112**
**EP - A - 0 022 531**
**DE - A - 2 645 496**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Regel, Erik, Bergerheide 72a, D-5600 Wuppertal 1 (DE)**
Erfinder: **Büchel, Karl Heinz, Prof.Dr., Dabringhausenerstrasse 42, D-5093 Burscheid (DE)**
Erfinder: **Lürssen, Klaus, Dr., August-Kierspel-Strasse 89, D-5060 Bergisch-Gladbach 2 (DE)**
Erfinder: **Frohberger, Paul-Ernst, Dr., Willi-Baumeister-Strasse 5, D-5090 Leverkusen 1 (DE)**

**EP 0 059 894 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue Triazolylalkylthioether, mehrere Verfahren zu ihrer Herstellung sowie ihre Verwendung als Pflanzenwachstumsregulatoren und Fungizide.

Es ist bereits bekanntgeworden, daß 4,4-Dimethyl-1-phenyl-2-triazolyl-1-penten-3-one und -ole eine gute fungizide Wirksamkeit besitzen (vergleiche die japanische Patentanmeldung J 53 150 661 und die DE-A 2 838 847). Die fungizide Wirkung dieser Verbindungen ist jedoch, insbesondere bei niedrigen Aufwandmengen und -konzentrationen, nicht immer voll befriedigend. Auch die pflanzenwachstumsregulierende Wirkung dieser Azolyl-Derivate ist nicht immer ausreichend.

Weiterhin ist bereits bekanntgeworden, daß 3-Cyclopropropyl-1-phenyl-2-triazolyl-1-propen-3-ole gute fungizide und pflanzenwachstumsregulierende Eigenschaften besitzen (vergleiche DE-A 3 010 560). Die Wirkung dieser Azolyl-Derivate läßt jedoch ebenfalls, insbesondere bei niedrigen Aufwandmengen und -konzentrationen, in manchen Fällen zu wünschen übrig.

Schließlich sind aus der DE-A 2 645 496, der EP-A 22 531 und der EP-A 112 zahlreiche Triazolyl-Derivate mit fungiziden und zum Teil auch pflanzenwuchsregulierenden Eigenschaften bekannt. Jedoch auch die Wirksamkeit dieser Stoffe ist nicht immer ausreichend.

Es wurden nun neue Triazolylalkyl-thioether der Formel (I)

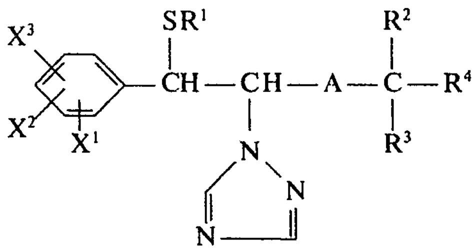

(I)

in welcher

A für die Ketogruppe oder die CH(OH)-Gruppierung steht,
$R^1$ für Alkyl mit 1 bis 4 Kohlenstoffatomen oder für gegebenenfalls durch Fluor, Chlor, Brom und/oder Alkyl mit 1 bis 4 Kohlenstoffatomen substituiertes Phenyl steht,
$R^2$ für Alkyl mit 1 bis 4 Kohlenstoffatomen steht,
$R^3$ für Alkyl mit 1 bis 4 Kohlenstoffatomen steht,
$R^4$ für Alkyl mit 1 bis 4 Kohlenstoffatomen, Halogenalkyl mit 1 bis 4 Kohlenstoffatomen und 1 bis 5 gleichen oder verschiedenen Halogenatomen oder Alkylthiomethyl mit 1 bis 4 Kohlenstoffatomen im Alkylteil steht,
$X^1$ für Wasserstoff, Fluor, Chlor oder Brom steht,
$X^2$ für Wasserstoff, Fluor oder Chlor steht und
$X^3$ für Wasserstoff oder Chlor steht,

sowie deren Säureadditions-Salze und Metallsalz-Komplexe gefunden.

Die Verbindungen der Formel (I) besitzen zwei asymmetrische Kohlenstoffatome; sie können deshalb in der erythro- wie in der threo-Form vorliegen. In beiden Fällen liegen sie vorwiegend als Racemate vor.

Weiterhin wurde gefunden, daß man die Triazolylalkyl-thioether der Formel (I), deren Säureadditions-Salze und Metallsalz-Komplexe erhält, wenn man

a) Vinyltriazolyl-Derivate der Formel (II)

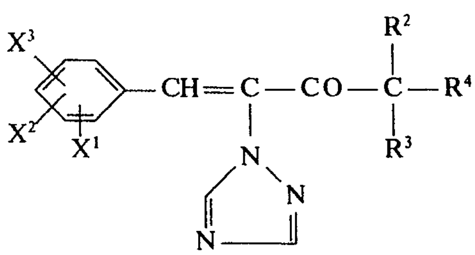

(II)

in welcher

$R^2$, $R^3$, $R^4$, $X^1$, $X^2$ und $X^3$ die oben angegebene Bedeutung haben,

mit Mercaptanen der Formel (III)

$R^1 - S - H$ (III)

in welcher

$R^1$ die oben angegebene Bedeutung hat,

in Gegenwart eines Verdünnungsmittels und gegebenenfalls in Gegenwart einer Base umsetzt, oder

b) Vinyltriazol-Derivate der Formel (II)

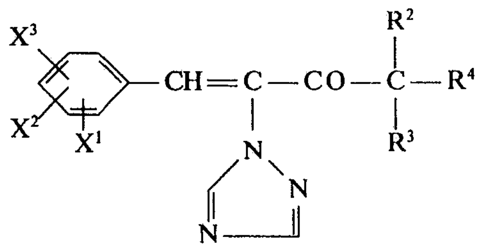

(II)

in welcher

$R^2$, $R^3$, $R^4$, $X^1$, $X^2$ und $X^3$ die oben angegebene Bedeutung haben,

mit Salzen von Isothioharnstoffen der Formel (IV)

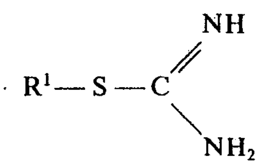

x Z (IV)

in welcher

$R^1$ die oben angegebene Bedeutung hat und
Z für das Äquivalent einer anorganischen Säure steht,

in Gegenwart eines Verdünnungsmittels und in Gegenwart einer Base umsetzt, oder

c) die nach den Verfahren (a) und (b) erhaltenen Triazolylalkyl-thioether-keto-Derivate der Formel (Ia)

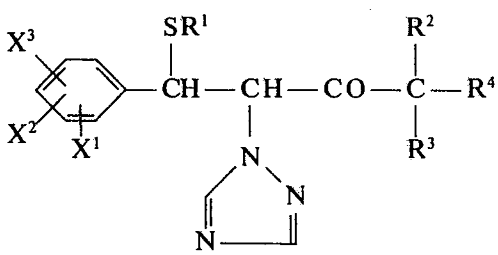

(Ia)

in welcher

$R^1$, $R^2$, $R^3$, $R^4$, $X^1$, $X^2$ und $X^3$ die oben angegebene Bedeutung haben,

in allgemein üblicher Weise reduziert,
und gegebenenfalls anschließend an die Verbindungen der Formel (I) eine Säure oder ein Metallsalz addiert.

Schließlich wurde gefunden, daß die neuen Triazolylalkyl-thioether der Formel (I) sowie deren Säureadditionssalze und Metallsalzkomplexe starke pflanzenwachstumsregulierende und starke fungizide Eigenschaften aufweisen.

Überraschenderweise zeigen die erfindungsgemäßen Verbindungen der Formel (I), deren Säureadditions-Salze und Metallsalz-Komplexe eine bessere pflanzenwachstumsregulierende und fungizide Wirkung als die aus dem Stand der Technik bekannten 4,4-Dimethyl-1-phenyl-2-triazolyl-1-penten-3-one und -ole sowie die 3-Cyclopropyl-1-phenyl-2-triazolyl-1-propen-3-ole, welche chemisch und wirkungsmäßig naheliegende Verbindungen sind. Die erfindungsgemäßen Wirkstoffe stellen somit eine Bereicherung der Technik dar.

Im übrigen übertreffen die erfindungsgemäßen Stoffe in ihrer pflanzenwuchsregulierenden Wirksamkeit auch die konstitutionell ähnlichsten Verbindungen, die aus der DE-A 2 645 496 und der EP-A 22 531 vorbekannt sind.

Die erfindungsgemäßen Triazolylalkyl-thioether sind durch die Formel (I) allgemein definiert. Bevorzugt sind diejenigen Verbindungen in denen

$R^1$ für geradkettiges oder verzweigtes Alkyl mit 1 bis 4 Kohlenstoffatomen oder für gegebenenfalls durch Fluor, Chlor und/oder geradkettiges oder verzweigtes Alkyl mit 1 bis 4 Kohlenstoffatomen substituiertes Phenyl steht,
$R^2$ für Methyl oder Ethyl steht,
$R^3$ für Methyl steht,
$R^4$ für Methyl, durch Fluor, Chlor und/oder Brom substituiertes Methyl oder Alkylthiomethyl mit 1 oder 2 Kohlenstoffatomen im Alkylteil steht,
$X^1$ für Wasserstoff, Fluor oder Chlor steht,
$X^2$ für Wasserstoff, Fluor oder Chlor steht und
$X^3$ für Wasserstoff oder Chlor steht.

Bevorzugt sind außerdem Säureadditions-Salze von Verbindungen der Formel (I), in denen A für die Ketogruppe oder für die CH(OH)-Gruppierung steht und $R^1$, $R^2$, $R^3$, $R^4$, $X^1$, $X^2$ und $X^3$ die vorzugsweise genannten Bedeutungen haben. Besonders bevorzugt sind dabei solche Säureadditions-Salze, die durch Addition von Halogenwasserstoffsäure, wie zum Beispiel Chlorwasserstoffsäure und Bromwasserstoffsäure, insbesondere Chlorwasserstoffsäure, ferner Phosphorsäure, Salpetersäure, Schwefelsäure, mono- und bifunktionelle Carbonsäuren und Hydroxycarbonsäuren, wie zum Beispiel Essigsäure, Maleinsäure, Bernsteinsäure, Fumarsäure, Weinsäure, Zitronensäure, Salicylsäure, Sorbinsäure, Milchsäure, sowie Sulfonsäuren, wie zum Beispiel p-Toluolsulfonsäure und 1,5-Naphthalindisulfonsäure entstehen.

Bevorzugt sind schließlich auch Metallsalzkomplexe von Verbindungen der Formel (I), in denen A für die Ketogruppe oder für die CH(OH)-Gruppierung steht und $R^1$, $R^2$, $R^3$, $R^4$, $X^1$, $X^2$ und $X^3$ die vorzugsweise genannten Bedeutungen haben. Besonders bevorzugt sind dabei solche Metallsalz-Komplexe, die als Kationen Metalle der II. bis IV. Hauptgruppe oder der I. und II. oder IV. bis VIII. Nebengruppe des Periodensystems der Elemente enthalten, wobei Kupfer, Zink, Mangan, Magnesium, Zinn, Eisen und Nickel beispielhaft genannt seien.

Anionen dieser Metallsalz-Komplexe sind vorzugsweise solche, die sich von Halogenwasserstoffsäuren, insbesondere Chlorwasserstoffsäure und Bromwasserstoffsäure, ferner von Phosphorsäure, Salpetersäure und Schwefelsäure ableiten.

Verwendet man beispielsweise 4,4-Dimethyl-1-(4-fluorphenyl)-2-(1,2,4-triazol-1-yl)-1-penten-3-on und Thiophenol als Ausgangsstoffe, so kann der Reaktionsablauf durch das folgende Formelschema wiedergegeben werden (Verfahren a):

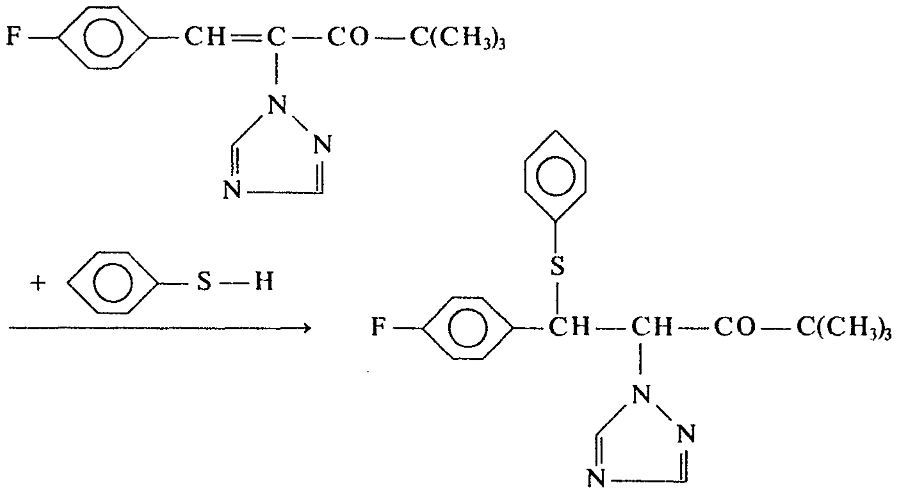

Verwendet man beispielsweise 1-(4-Chlorphenyl)-4,4-di-methyl-2-(1,2,4-triazol-1-yl)-1-penten-3-on und S-Methyl-isothioharnstoff-sulfat als Ausgangsstoffe, so kann der Reaktionsablauf durch das folgende Formelschema wiedergegeben werden (Verfahren b):

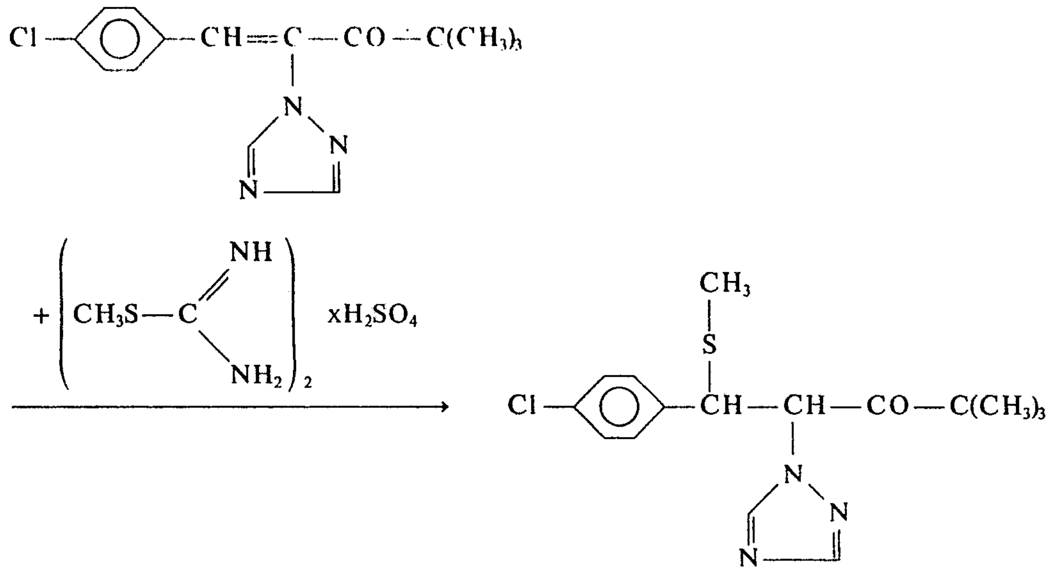


Verwendet man beispielsweise 1-(4-Chlorphenyl)-4,4-dimethyl-1-methylthio-2-(1,2,4-triazol-1-yl)-pentan-3-on und Natriumborhydrid als Ausgangsstoffe, so kann der Reaktionsablauf durch das folgende Formelschema wiedergegeben werden (Verfahren c):

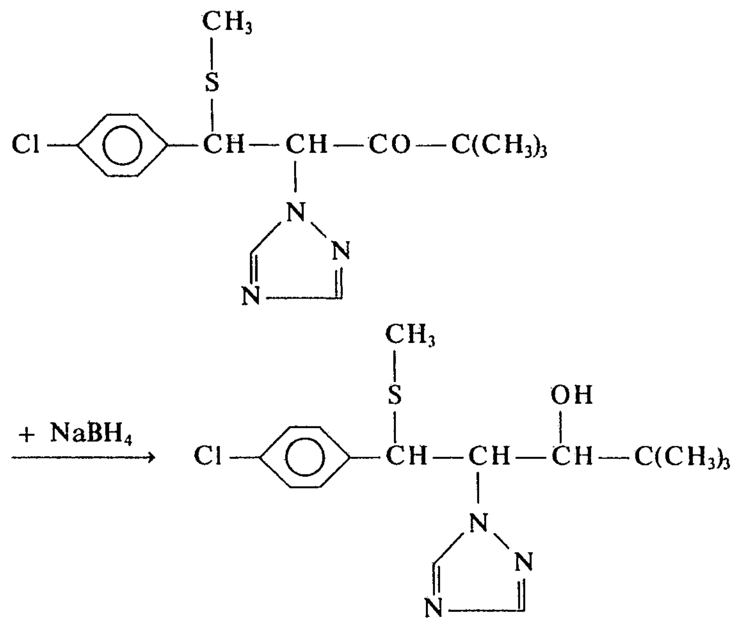


Die bei der Durchführung der erfindungsgemäßen Verfahren (a) und (b) als Ausgangsstoffe benötigten Vinyltriazol-Derivate sind durch die Formel (II) allgemein definiert. In dieser Formel haben $R^2$, $R^3$, $R^4$, $X^1$, $X^2$ und $X^3$ vorzugsweise diejenigen Bedeutungen, die bereits im Zusammenhang mit der Beschreibung der erfindungsgemäßen Stoffe der Formel (I) vorzugsweise für diese Reste genannt wurden.

Die Vinyltriazolyl-Derivate der Formel (II) sind teilweise bekannt (vgl. JP-A 53 130 661; DE-A 2 838 847, DE-A 3 010 560, DE-A 2 929 602 und DE-A 3 000 643); teilweise sind sie Gegenstand der deutschen Patentanmeldungen, die als DE-A 3 025 242, DE-A 3 028 330 und DE-A 3 044 802 veröffentlicht wurden. Sie können nach den dort angegebenen Verfahren erhalten werden, indem man z. B. entsprechende Ketone mit den entsprechenden Benzaldehyden in üblicher Weise in Gegenwart eines inerten organischen Lösungsmittels, wie beispielsweise Toluol, und in Gegenwart eines Katalysators, wie beispielsweise Piperidinacetat, bei Temperaturen zwischen 20 und 160°C, insbesondere bei der Siedetemperatur des jeweiligen Lösungsmittels, umsetzt.

Die Vinyltriazolyl-Derivate der Formel (II) kommen in den geometrischen Isomeren E (trans) und Z (cis) vor. Bei der E,Z-Nomenklatur werden die an der Doppelbindung stehenden Substituenten nach der Cahn-Ingold-Prelog-Regel nach abnehmender Priorität eingeordnet. Stehen die bevorzugten Substituenten auf derselben Seite der Doppelbindung, liegt die Konfiguration Z (abgeleitet von zusammen) vor, stehen sie auf entgegengesetzter Seite, liegt die Konfiguration E (abgeleitet von entgegen) vor.

Ein eindeutiges Charakterisierungsmerkmal für die beiden geometrischen Isomeren ist die $H^1$-Kernresonanz der zwei Triazol-Protonen. Die Differenz der Shiftwerte für diese beiden Protonen ist in den E-Formen ungefähr doppelt so groß wie in den entsprechenden Z-Formen.

Die Vinyltriazolyl-Derivate der Formel (II) können sowohl als E/Z-Isomerengemisch, als auch in Form der einzelnen E- und Z-Isomeren eingesetzt werden.

Die außerdem für das erfindungsgemäße Verfahren (a) als Ausgangsstoffe benötigten Mercaptane sind durch die Formel (III) allgemein definiert. In dieser Formel steht $R^1$ vorzugsweise für diejenigen Reste, die bereits im Zusammenhang mit der Beschreibung der erfindungsgemäßen Stoffe der Formel (I) vorzugsweise für diesen Substituenten genannt wurden.

Die Mercaptane der Formel (III) sind allgemein bekannte Verbindungen der organischen Chemie.

Die außerdem für das erfindungsgemäße Verfahren (b) als Ausgangsstoffe benötigten Salze von Isothioharnstoffen sind durch die Formel (IV) allgemein definiert. In dieser Formel steht $R^1$ vorzugsweise für diejenigen Reste, die bereits im Zusammenhang mit der Beschreibung der erfindungsgemäßen Stoffe der Formel (I) vorzugsweise für diesen Substituenten genannt wurden. Z steht vorzugsweise für anorganische Sauerstoffsäuren, wie beispielsweise Schwefelsäure, Phosphorsäure oder Salpetersäure, sowie für Halogenwasserstoffsäure, wie z. B. Chlor- und Bromwasserstoffsäure.

Die Isothioharnstoffe der Formel (IV) sind allgemein bekannte Verbindungen der organischen Chemie.

Die für das erfindungsgemäße Verfahren (c) als Ausgangsstoffe benötigten Triazolylalkyl-thioether-keto-Derivate der Formel (Ia) sind erfindungsgemäße Verbindungen.

Als Verdünnungsmittel kommen für das erfindungsgemäße Verfahren (a) vorzugsweise inerte organische Lösungsmittel in Frage. Hierzu gehören vorzugsweise Ketone, wie Aceton und Methylethylketon; Alkohole, wie Methanol, Ethanol und Isopropanol; Nitrile, wie Acetonitril und Propionitril; aromatische Kohlenwasserstoffe, wie Benzol, Toluol oder Xylol; sowie Ether, wie Diethylether oder Tetrahydrofuran.

Das erfindungsgemäße Verfahren (a) wird gegebenenfalls in Gegenwart einer Base durchgeführt. Hierfür kommen vorzugsweise in Frage: Alkalimetallalkoholate, wie z. B. Natrium- oder Kaliummethylat bzw. -ethylat; Alkalimetallamide, wie z. B. Natrium- oder Kaliumamid; sowie Alkalimetallhydride, wie z. B. Natriumhydrid.

Die Reaktionstemperaturen können beim Verfahren (a) in einem größeren Bereich variiert werden. Im allgemeinen arbeitet man zwischen 0 und 120°C, vorzugsweise bei 20 bis 80°C.

Bei der Durchführung des erfindungsgemäßen Verfahrens (a) setzt man auf 1 Mol Vinyltriazolyl-Derivat der Formel (II) vorzugsweise 1 bis 2 Mol Mercaptan der Formel (III) sowie gegebenenfalls katalytische bis molare Mengen an Base ein. Die Isolierung der Endprodukte der Formel (I) erfolgt in allgemein üblicher und bekannter Art und Weise.

Als Verdünnungsmittel kommen für das erfindungsgemäße Verfahren (b) vorzugsweise inerte organische Lösungsmittel in Frage. Hierzu gehören vorzugsweise die bereits beim Verfahren (a) vorzugsweise genannten Solventien.

Das erfindungsgemäße Verfahren (b) wird in Gegenwart einer Base durchgeführt. Man kann alle üblicherweise verwendbaren anorganischen und organischen Basen zugeben, wie Alkalicarbonate, beispielsweise Natriumcarbonat, Kaliumcarbonat oder Natriumhydrogencarbonat; oder wie niedere tertiäre Alkylamine, Cycloalkylamine oder Aralkylamine, wie Triethylamin, Dimethylbenzylamin oder Cyclohexylamin; sowie Alkalialkoholate, wie Natriummethylat oder -ethylat.

Die Reaktionstemperaturen können beim Verfahren (b) in einem größeren Bereich variiert werden. Im allgemeinen arbeitet man zwischen 20 und 150°C, vorzugsweise bei der Siedetemperatur des Lösungsmittels.

Bei der Durchführung des erfindungsgemäßen Verfahrens (b) setzt man auf 1 Mol Vinyltriazolyl-Derivate der Formel (II) vorzugsweise die äquivalente Menge Isothioharnstoff der Formel (IV) sowie 1 bis 4 Mol Base ein. Die Isolierung der Endprodukte der Formel (I) erfolgt in allgemein üblicher und bekannter Weise.

Die erfindungsgemäße Reduktion gemäß Verfahren (c) erfolgt in üblicher Weise, z. B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung (c) polare organische Lösungsmittel in Frage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei −10 bis +30°C, vorzugsweise bei −10 bis 20°C durchgeführt. Hierzu setzt man auf 1 Mol des Ketons der Formel (Ia) etwa 1 Mol eines komplexen Hydrids, wie Natriumborhydrid, Calciumborhydrid oder Lithiumalanat, ein. Die Isolierung der erfindungsgemäßen Verbindungen erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, in

Frage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des entsprechenden Ketons der Formel (Ia) etwa 1 bis 2 Mol Aluminiumisopropylat ein. Die Isolierung der erfindungsgemäßen Verbindungen erfolgt in üblicher Weise.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen von Verbindungen der Formel (I) kommen vorzugsweise Salze von denjenigen Anionen und Kationen in Betracht, die bereits im Zusammenhang mit der Beschreibung der erfindungsgemäßen Metallsalz-Komplexe als bevorzugt genannt werden.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindung der Formel (I). Man kann Metallsalz-Komplexe in bekannter Weise, z. B. durch Abfiltrieren, Isolieren und gegebenenfalls durch Umkristallisation reinigen.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkung auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrädern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens (»Lagerns«) der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es höhere Düngemengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leicht bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z. B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden (»Ausdünnung«), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z. B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z. B. gegen den Erreger des Gersten- bzw. des Getreidemehltaus (Erysiphe graminis); sowie zur Bekämpfung von Reiskrankheiten, wie z. B. Pellicularia sasakii.

Hervorzuheben ist, daß die erfindungsgemäßen Wirkstoffe nicht nur eine protektive Wirkung entfalten, sondern auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

In entsprechenden Aufwandmengen zeigen die erfindungsgemäßen Verbindungen auch herbizide Wirkungen.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommem im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol, oder Glykol sowie deren Ether

und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid. Als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z. B. nichtionogene und anionische Emulgatoren, wie Polyoxyethylen-Fettsäureester, Polyoxyethylen-Fettalkoholether, z. B. Alkylarylpolyglykol-ether, Alkylsulfonate, Alkylsulfate, Arylsulfonate sowie Eiweißhydrolysate. Als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid,Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen Schäume, Suspensionen, Spritzpulver, Pasten, lösliche Pulver, Stäubemittel und Granulate, angewendet werden. Die Anwendung geschieht in üblicher Weise, z. B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach klimatischen und vegetativen Gegebenheiten richtet.

Auch beim Einsatz der erfindungsgemäßen Stoffe als Fungizide kann die Aufwandmenge je nach Art der Applikation in einem größeren Bereich variiert werden. So liegen die Wirkungskonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50 g je kg Saatgut, vorzugsweise 0,01 bis 10 g, benötigt. Bei der Behandlung des Bodens sind Wirkstoffkonzentrationen von 0,00001 bis 0,1 Gew.-%, vorzugsweise von 0,0001 bis 0,02%, am Wirkungsort erforderlich.

Herstellung und Verwendung der erfindungsgemäßen Stoffe gehen aus den nachfolgenden Beispielen hervor.

## Herstellungsbeispiele

### Beispiel 1

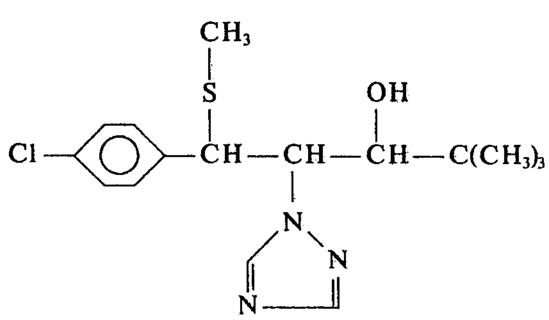

(Verfahren c)

Zu einer Suspension von 33,75 g (0,1 Mol) 1-(4-Chlor-phenyl)-4,4-dimethyl-1-methylthio-2-(1,2,4-triazol-1-yl)-pentan-3-on (Beispiel 2) und 7,6 g (0,0683 Mol) wasserfreiem Calciumchlorid in 200 ml Isopropanol wird bei −5°C eine Lösung von 2,7 g (0,0703 Mol) Natriumboranat in 25 ml Wasser zugetropft. Das Gemisch wird 15 Stunden nachgerührt. Nach Zugabe von 20 ml Aceton wird das Gemisch im Vakuum eingedampft. Der Rückstand wird in Wasser eingerührt und mit Methylenchlorid mehrmals ausgeschüttelt. Die Methylenchlorid-Phase wird über Natriumsulfat getrocknet, filtriert und im Vakuum eingedampft. Man erhält 32,6 g (96% der Theorie) 1-(4-Chlorphenyl)-4,4-dimethyl-1-methylthio-2-(1,2,4-triazol-1-yl)-pentan-3-ol vom Schmelzpunkt 58–60°C.

Beispiel 2

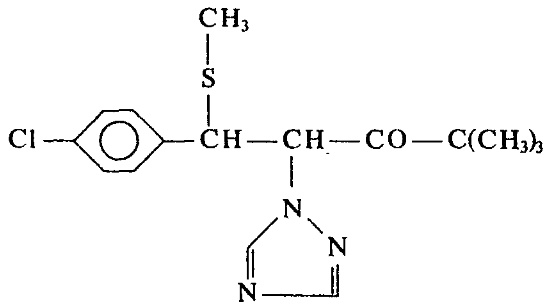

(Verfahren a)

10 g (0,2 Mol) Methylmercaptan werden in eine Lösung von 0,5 g (9 mMol) Natriummethylat in 300 ml Ethanol eingeleitet. In diese Lösung werden 47 g (0,16 Mol) 1-(4-Chlorphenyl)-4,4-dimethyl-2-(1,2,4-triazol-1-yl)-1-penten-3-on (E-Isomeres) eingetragen. Die anfangs klare Lösung wandelt sich bald zu einem dicken Kristallbrei um. Die Kristalle werden abgesaugt, mit Diisopropylether und mit Ethanol gewaschen und getrocknet. Man erhält 36 g (66,6% der Theorie) 1-(4-Chlorphenyl)-4,4-dimethyl-1-methylthio-2-(1,2,4-triazol-1-yl)-pentan-3-on vom Schmelzpunkt 145°C.

(Verfahren b)

289 g (1 Mol) 1-(4-Chlorphenyl)-4,4-dimethyl-2-(1,2,4-triazol-1-yl)-1-penten-3-on (E,Z-Isomerengemisch) und 139 g (0,5 Mol) S-Methylisothioharnstoff-sulfat werden in 2,5 l Ethanol suspendiert und unter Rühren portionsweise mit 336 g (4 Mol) Natriumhydrogencarbonat versetzt. Das Gemisch wird 4 Stunden unter Rückfluß erhitzt, auf Raumtemperatur abgekühlt und auf Wasser gegossen. Die organischen Anteile werden mit Methylenchlorid extrahiert. Die organische Phase wird über Natriumsulfat getrocknet, filtriert und eingedampft. Das resultierende Öl wird durch Verrühren mit Diisopropylether zur Kristallisation gebracht und die Kristalle werden aus Ethanol umkristallisiert. Man erhält 42 g (12% der Theorie) 1-(4-Chlorphenyl)-4,4-dimethyl-1-methylthio-2-(1,2,4-triazol-1-yl)-pentan-3-on vom Schmelzpunkt 145°C.

Herstellung des Ausgangsproduktes

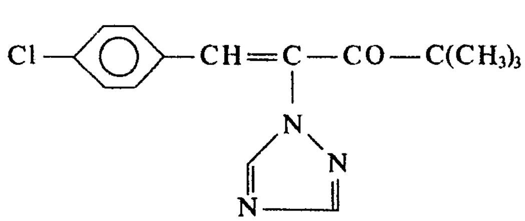

167 g (1 Mol) 3,3-Dimethyl-1-(1,2,4-triazol-1-yl)-butan-2-on und 140,5 g (1 Mol) 4-Chlorbenzaldehyd werden in 700 ml Toluol gelöst und mit 1,2 g (0,02 Mol) Eisessig und 1,8 ml (0,02 Mol) Piperidin versetzt. Das Reaktionsgemisch wird 40 Stunden auf 120°C erhitzt, wobei das Reaktionswasser laufend abgetrennt wird. Danach wird das Reaktionsgemisch im Vakuum eingeengt und das zurückbleibende Öl destilliert (Siedebereich 130–160°C/0,04 Torr). Das Destillat wird mit Ethanol verrührt, wobei sich Kristalle abscheiden. Diese werden abgesaugt und getrocknet. Man erhält so 35 g (12% der Thoerie) 1-(4-Chlorphenyl)-4,4-dimethyl-2-(1,2,4-triazol-1-yl)-1-penten-3-on (E-Isomeres) vom Schmelzpunkt

110°C.

Die Ethanol-Mutterlauge wird eingeengt und das zurückbleibende Öl in Diisopropylether gelöst, wobei es zur Auskristallisation kommt. Nach Umkristallisation aus Diisopropylether erhält man 25,5 g (8,8% der Theorie) 1-(4-Chlorphenyl)-4,4-dimethyl-2-(1,2,4-triazol-1-yl)-1-penten-3-on (Z-Isomeres) vom Schmelzpunkt 82°C.

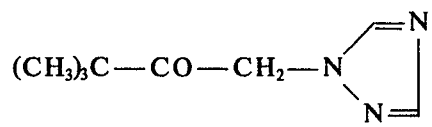

138 g (2 Mol) 1,2,4-Triazol werden bei Raumtemperatur portionsweise zu 276,4 g (2 Mol) gemahlenem Kaliumcarbonat und 269,2 g (2 Mol) α-Chlorpinakolin in 500 ml Aceton gegeben, wobei die Innentemperatur bis zur Siedehitze ansteigt. Man läßt 5 Stunden unter Rückfluß rühren und kühlt dann auf Raumtemperatur ab. Das Reaktionsgemisch wird filtriert und das Filtrat durch Abdestillieren des Lösungsmittels im Vakuum eingeengt. Der ölige Rückstand kristallisiert nach Zugabe von Benzin. Man erhält 240,8 g (72% der Theorie) 3,3-Dimethyl-1-(1,2,4-triazol-1-yl)-butan-2-on vom Schmelzpunkt 62–64°C.

Beispiel 3

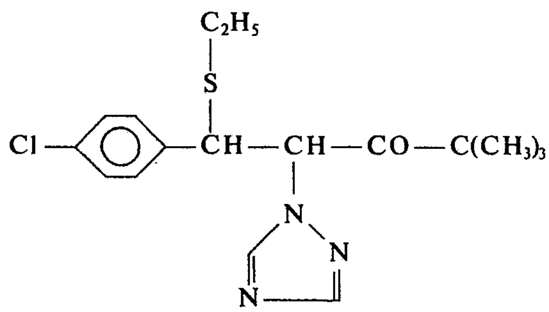

(Verfahren a)

28,9 g (0,1 Mol) 1-(4-Chlorphenyl)-4,4-dimethyl-2-(1,2,4-triazol-1-yl)-1-penten-3-on (E-Isomeres) werden in eine Suspension von 8,4 g (0,1 Mol) Natriummethylmercaptid in 250 ml Ether eingetragen. Das Gemisch wird 20 Stunden gerührt und dann im Vakuum eingedampft. Der Rückstand wird in Wasser gegeben und mit Methylenchlorid extrahiert. Die Methylenchloridlösung wird über Natriumsulfat getrocknet, filtriert und im Vakuum eingedampft. Der kristalline Rückstand wird mit Diisopropylether verrührt und abgesaugt. Man erhält 14,9 g (44,9% der Theorie) 1-(4-Chlorphenyl)-4,4-dimethyl-1-ethylthio-2-(1,2,4-triazol-1-yl)-pentan-3-on vom Schmelzpunkt 154°C.

Beispiel 4

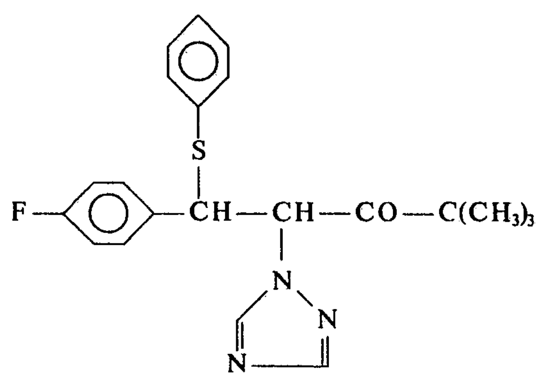

(Verfahren a)

27,3 g (0,1 Mol) 4,4-Dimethyl-1-(4-fluorphenyl)-2-(1,2,4-triazol-1-yl)-1-penten-3-on (E,Z-Isomerengemisch) und 11 g (0,1 Mol) Thiophenol werden in 100 ml Ethanol bei Raumtemperatur verrührt. Nach

1 Stunde werden die ausgeschiedenen Kristalle abgesaugt und getrocknet. Man erhält 34,7 g (91% der Theorie) 4,4-Dimethyl-1-(4-fluorphenyl)-1-phenylthio-2-(1,2,4-triazol-1-yl)-pentan-3-on vom Schmelzpunkt 175°C.

Beispiel 5

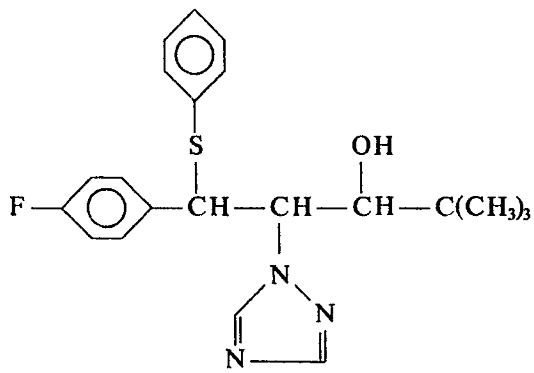

(Verfahren c)

9,5 g (25 mMol) 4,4-Dimethyl-1-(4-fluorphenyl)-1-phenylthio-2-(1,2,4-triazol-1-yl)-pentan-3-on (Beispiel 4) werden in 150 ml Isopropylalkohol suspendiert und portionsweise mit 0,5 g (12,5 mMol) Natriumborhydrid versetzt. Nach 2 Tagen wird das Gemisch im Vakuum eingedampft und mit verdünnter Essigsäure zersetzt. Die organische Phase wird in Methylenchlorid aufgenommen, mit Wasser gewaschen, über Natriumsulfat getrocknet, filtriert und im Vakuum eingedampft. Der kristalline Rückstand wird mit Diisopropylether verrührt; die Kristalle werden abgesaugt und getrocknet. Man erhält 7,5 g (77,9% der Theorie) 4,4-Dimethyl-1-(4-fluorphenyl) -1-phenylthio-2-(1,2,4-triazol-1-yl)-pentan-3-ol vom Schmelzpunkt 171°C.

Beispiel 6

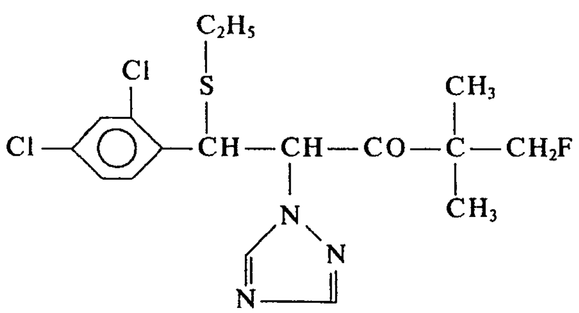

(Verfahren a)

Zu einer Suspension von 3,19 g (0,059 Mol) Natriummethylat in 200 ml Diethylether werden 4,4 ml (0,059 Mol) Ethylmercaptan zugegeben. Nach 2 Stunden werden 20,2 g (0,059 Mol) 1-(2,4-Dichlorphenyl)-4,4-dimethyl-5-fluor-2-(1,2,4-triazol-1-yl)-1-penten-3-on (E-Isomeres) eingetragen. Das Gemisch wird 18 Stunden bei 25°C gerührt und anschließend im Vakuum eingedampft. Der Rückstand wird in Wasser gegeben und mit Methylenchlorid extrahiert. Die Methylenchloridlösung wird über Natriumsulfat getrocknet, filtriert und im Vakuum eingedampft. Das verbleibende Öl wird durch Verrühren mit Diisopropylether zur Kristallisation gebracht. Man erhält 13 g (54,6% der Theorie) 1-(2,4-Dichlorphenyl)-4,4-dimethyl-1-ethylthio-5-fluor-2-(1,2,4-triazol-1-yl)-pentan-3-on vom Schmelzpunkt 130°C.

Beispiel 7

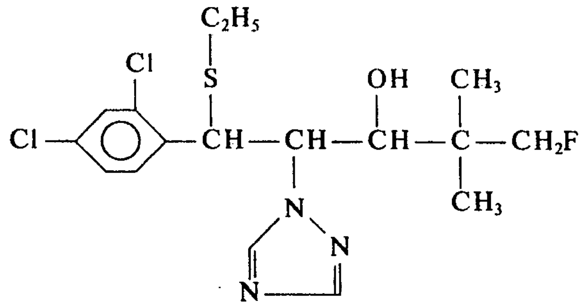

(Verfahren c)

Eine Lösung von 0,42 g (11 mMol) Natriumboranat in 20 ml Wasser wird unter Rühren in eine Suspension von 6,4 g (15,8 mMol) 1-(2,4-Dichlorphenyl)-4,4-dimethyl-1-ethylthio-5-fluor-2-(1,2,4-triazol-1-yl)-pentan-3-on (Beispiel 6) und 1,2 g (10,8 mMol) Calciumchlorid in 150 ml Isopropanol eingetropft. Das Gemisch wird 18 Stunden bei 0°C gerührt und anschließend tropfenweise mit 20 ml Aceton versetzt. Das Gemisch wird im Vakuum eingedampft und der Rückstand wird mit verdünnter Essigsäure zersetzt. Die organische Phase wird in Methylenchlorid gelöst, mit Wasser gewaschen, über Natriumsulfat getrocknet, filtriert und im Vakuum eingedampft. Das verbleibende Öl wird mit Diisopropylether verrührt. Man erhält 3,9 g (60% der Theorie) 1-(2,4-Dichlorphenyl)-4,4-dimethyl-1-ethylthio-5-fluor-2-(1,2,4-triazol-1-yl)-pentan-3-ol vom Schmelzpunkt 182°C.

In entsprechender Weise und gemäß den angegebenen Verfahren werden die in der folgenden Tabelle 1 aufgeführten Verbindungen der Formel (I)

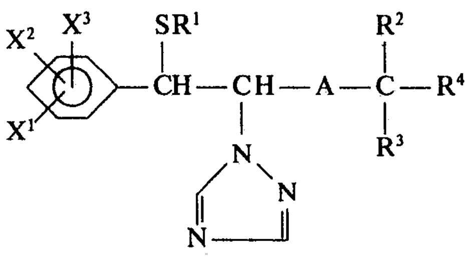

(I)

erhalten:

**Tabelle 1**

| Beispiel Nr. | $X^1$ | $X^2$ | $X^3$ | $R^1$ | A | $R^2$ | $R^3$ | $R^4$ | Schmelzpunkt (°C) |
|---|---|---|---|---|---|---|---|---|---|
| 8 | 4-Cl | H | H | 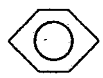 | CO | $CH_3$ | $CH_3$ | $CH_3$ | 184 |
| 9 | 4-Cl | H | H | 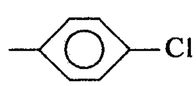 | CO | $CH_3$ | $CH_3$ | $CH_3$ | 132 |
| 10 | 4-F | H | H | 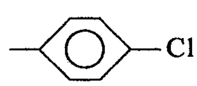 | CO | $CH_3$ | $CH_3$ | $CH_3$ | 149 |
| 11 | 3-Cl | H | H | 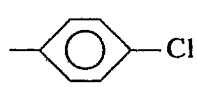 | CO | $CH_3$ | $CH_3$ | $CH_3$ | 152 |
| 12 | 4-Cl | H | H | $C_2H_5$ | CO | $CH_3$ | $CH_3$ | $CH_2SC_2H_5$ | $n_D^{20}$: 1,5578 |
| 13 | 4-Cl | H | H | 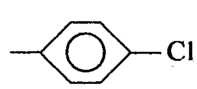 | CO | $CH_3$ | $CH_3$ | $CH_2F$ | 129 |

Fortsetzung

| Beispiel Nr. | $X^1$ | $X^2$ | $X^3$ | $R^1$ | A | $R^2$ | $R^3$ | $R^4$ | Schmelzpunkt (°C) |
|---|---|---|---|---|---|---|---|---|---|
| 14 | 4-Cl | H | H | 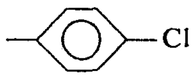 | CO | $CH_3$ | $CH_3$ | $CH_2Cl$ | 146 |
| 15 | 2-Cl | 4-Cl | H | $C_2H_5$ | CH(OH) | $CH_3$ | $CH_3$ | $CH_3$ | 180 |
| 16 | 4-F | H | H | 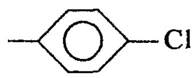 | CH(OH) | $CH_3$ | $CH_3$ | $CH_3$ | 201 |
| 17 | 4-Cl | H | H | $C_4H_9$ | CO | $CH_3$ | $CH_3$ | $CH_3$ | 80 |
| 18 | 4-Cl | H | H | $C_2H_5$ | CH(OH) | $CH_3$ | $CH_3$ | $CH_3$ | 170 |
| 19 | 4-F | H | H | $CH_3$ | CH(OH) | $CH_3$ | $CH_3$ | $CH_2Cl$ | viskoses Öl |
| 20 | 4-Cl | H | H | $C_2H_5$ | CH(OH) | $CH_3$ | $CH_3$ | $CH_2SC_2H_5$ | 96 |
| 21 | 4-Cl | H | H | $C_4H_9$ | CH(OH) | $CH_3$ | $CH_3$ | $CH_3$ | 165 |
| 22 | 4-Cl | H | H | 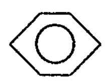 | CH(OH) | $CH_3$ | $CH_3$ | $CH_3$ | 180 |
| 23 | 4-Cl | H | H | 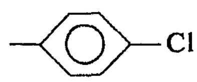 | CH(OH) | $CH_3$ | $CH_3$ | $CH_2Cl$ | 88 |
| 24 | 3-Cl | H | H | 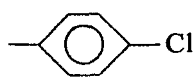 | CH(OH) | $CH_3$ | $CH_3$ | $CH_3$ | 148 |

## Anwendungsbeispiele

In den nachfolgenden Anwendungsbeispielen werden die nachstehend angegebenen Verbindungen als Vergleichssubstanzen eingesetzt:

(A)

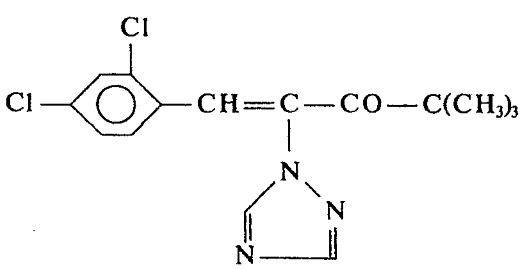

(B)

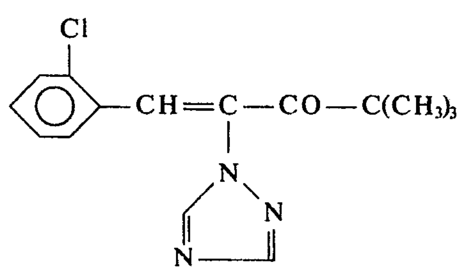

(C) 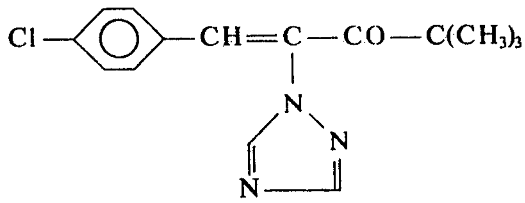

(D) 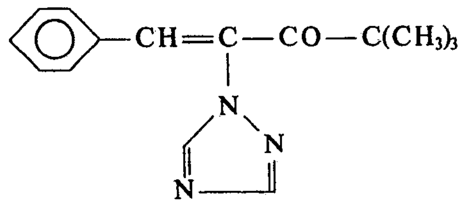

(E) 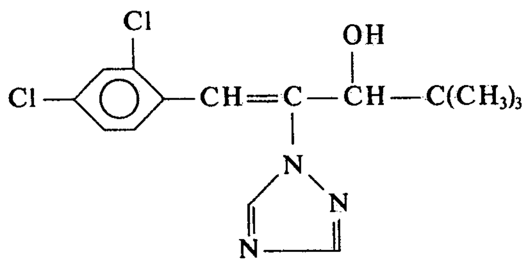

(F) 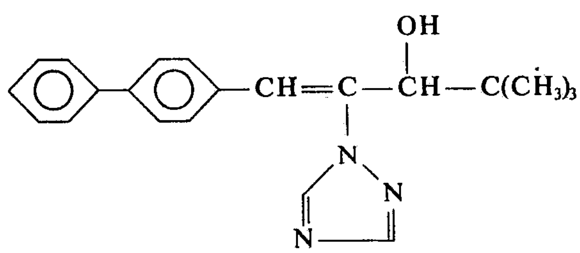

(G) 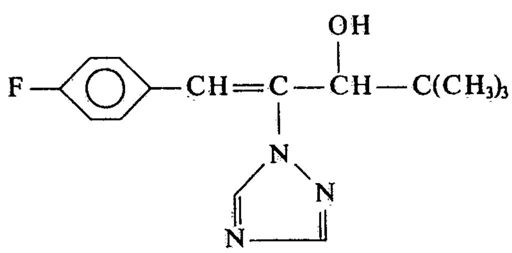

(H) 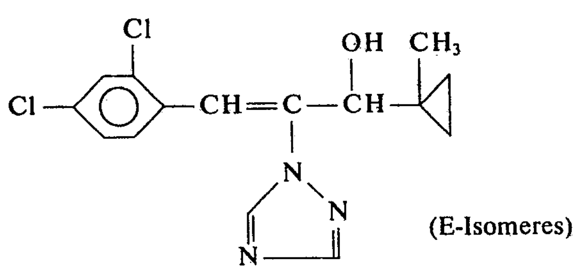

(E-Isomeres)

(I) 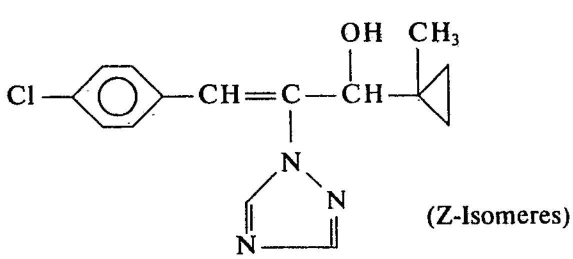

(Z-Isomeres)

## Beispiel A

### Wuchshemmung bei Gras (Festuca pratensis)

Lösungsmittel: 30 Gewichtsteile Dimethylformamid
Emulgator: 1 Gewichtsteil Polyoxyethylen-Sorbitanmonolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen mit den Wirkstoffzubereitungen tropfnaß besprüht. Nach 3 Wochen wird der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Der erfindungsgemäße Wirkstoff 1 zeigt in diesem Test eine bessere Wuchshemmung als die aus dem Stand der Technik bekannten Verbindungen (A), (B), (C), (E), (F), (G), (H) und (I).

## Beispiel B

### Wuchshemmung bei Gerste

Lösungsmittel: 30 Gewichtsteile Dimethylformamid
Emulgator: 1 Gewichtsteil Polyoxyethylen-Sorbitanmonolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstenpflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Der erfindungsgemäße Wirkstoff (1) zeigt in diesem Test eine bessere Wuchshemmung als die aus dem Stand der Technik bekannten Verbindungen (A), (B), (C), (G), (H) und (I).

## Beispiel C

### Wuchsbeeinflussung bei Zuckerrüben

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitungen besprüht. Nach 14 Tagen wird der Zuwachs der Pflanzen gemessen und die Wuchsbeeinflussung in Prozent des Zuwachses der Kontrollpflanzen berechnet. Es bedeuten 0% Wuchsbeeinflussung ein Wachstum entsprechend dem der Kontrollpflanzen. Negative Werte kennzeichnen eine Wuchshemmung, positive Werte eine Wuchsförderung gegenüber den Kontrollpflanzen.

Die erfindungsgemäßen Wirkstoffe 1, 2 und 8 zeigen in diesem Test eine bessere Wuchsbeeinflussung als die aus dem Stand der Technik bekannten Verbindungen (A), (B), (D) und (F).

## Beispiel D

### Wuchshemmung bei Sojabohnen

Lösungsmittel: 30 Gewichtsteile Dimethylformamid
Emulgator: 1 Gewichtsteil Polyoxyethylen-Sorbitanmonolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die erfindungsgemäßen Wirkstoffe 1 und 9 zeigen in diesem Test eine bessere Wirkung als die aus dem Stand der Technik bekannten Verbindungen (A), (B), (D), (F), (H) und (I).

## Beispiel E

### Wuchshemmung bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit der angegebenen Menge Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die erfindungsgemäßen Wirkstoffe 1, 4 und 10 zeigen in diesem Test eine bessere Wuchshemmung als die aus dem Stand der Technik bekannten Verbindungen (A), (B), (D), (E), (F) und (G).

## Beispiel F

### Erysiphe-Test (Gerste) / protektiv /

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber den aus dem Stand der Technik bekannten Verbindungen (A) und (B) zeigen bei diesem Test z. B. die Verbindungen gemäß folgender Herstellungsbeispiele: 2, 1, 9, 10, 4 und 16.

## Beispiel G

### Gerstenmehltau-Test (Erysiphe graminis var. hordei)/ systemisch (pilzliche Getreidesproßkrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 · 12 Korn in Blumentöpfe 2 cm tief in ein Gemisch aus einem Volumenteil Fruhstorfer Einheitserde und einem Volumenteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen

von Erysiphe graminis var. hordei bestäubt und bei 21–22°C und 80–90% rel. Luftfeuchte und 16stündiger Belichtung weiter kultiviert. Innerhalb von 6 Tagen bilden sich an den Blättern die typischen Mehltaupusteln aus.

Der Befallsgrad wird in Prozent des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Eine deutliche Überlegenheit in der Wirksamkeit gegenüber den aus dem Stand der Technik bekannten Verbindungen (A) und (F) zeigt bei diesem Test z. B. die Verbindung gemäß Herstellungsbeispiel 1.

**Patentansprüche**

1. Triazolylalkyl-thioether der Formel (I)

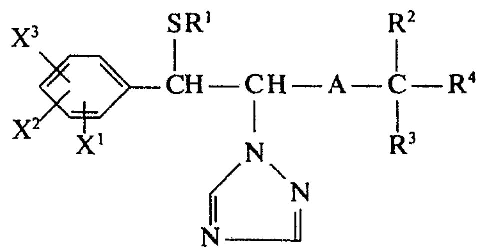

(I)

in welcher

A für die Ketogruppe oder die CH(OH)-Gruppierung steht,
$R^1$ für Alkyl mit 1 bis 4 Kohlenstoffatomen oder für gegebenenfalls durch Fluor, Chlor, Brom und/oder Alkyl mit 1 bis 4 Kohlenstoffatomen substituiertes Phenyl steht,
$R^2$ für Alkyl mit 1 bis 4 Kohlenstoffatomen steht,
$R^3$ für Alkyl mit 1 bis 4 Kohlenstoffatomen steht,
$R^4$ für Alkyl mit 1 bis 4 Kohlenstoffatomen, Halogenalkyl mit 1 bis 4 Kohlenstoffatomen und 1 bis 5 gleichen oder verschiedenen Halogenatomen oder Alkylthiomethyl mit 1 bis 4 Kohlenstoffatomen im Alkylteil steht,
$X^1$ für Wasserstoff, Fluor, Chlor oder Brom steht,
$X^2$ für Wasserstoff, Fluor oder Chlor steht und
$X^3$ für Wasserstoff oder Chlor steht,

sowie deren Säureadditions-Salze und Metallsalz-Komplexe.

2. Verfahren zur Herstellung von Triazolylalkyl-thioethern der Formel (I)

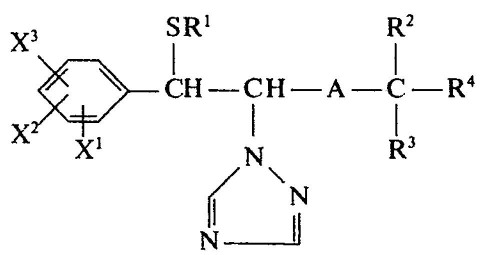

(I)

in welcher

A für die Ketogruppe oder die CH(OH)-Gruppierung steht,
$R^1$ für Alkyl mit 1 bis 4 Kohlenstoffatomen oder für gegebenenfalls durch Fluor, Chlor, Brom und/oder Alkyl mit 1 bis 4 Kohlenstoffatomen substituiertes Phenyl steht,
$R^2$ für Alkyl mit 1 bis 4 Kohlenstoffatomen steht,
$R^3$ für Alkyl mit 1 bis 4 Kohlenstoffatomen steht,
$R^4$ für Alkyl mit 1 bis 4 Kohlenstoffatomen, Halogenalkyl mit 1 bis 4 Kohlenstoffatomen und 1 bis 5 gleichen oder verschiedenen Halogenatomen oder Alkylthiomethyl mit 1 bis 4 Kohlenstoffatomen im Alkylteil steht,
$X^1$ für Wasserstoff, Fluor, Chlor oder Brom steht,
$X^2$ für Wasserstoff, Fluor oder Chlor steht und
$X^3$ für Wasserstoff oder Chlor steht,

sowie von deren Säureadditions-Salzen und Metallsalz-Komplexen, dadurch gekennzeichnet, daß man

a) Vinyltriazolyl-Derivate der Formel (II)

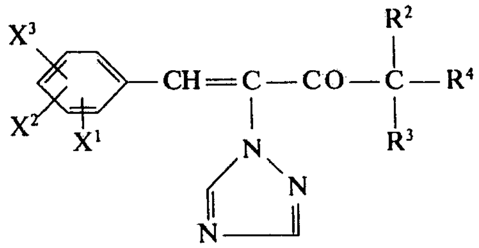

(II)

in welcher

$R^2$, $R^3$, $R^4$, $X^1$, $X^2$ und $X^3$ die oben angegebene Bedeutung haben,

mit Mercaptanen der Formel III

$R^1 - S - H$ (III)

in welcher

$R^1$ die oben angegebene Bedeutung hat,

in Gegenwart eines Verdünnungsmittels und gegebenenfalls in Gegenwart einer Base umsetzt, oder

b) Vinyltriazolyl-Derivate der Formel (II)

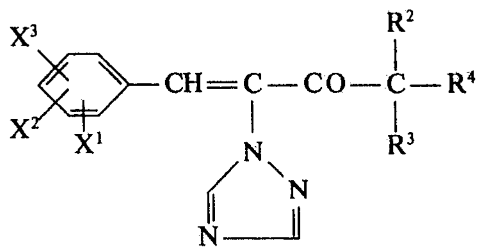

(II)

in welcher

$R^2$, $R^3$, $R^4$, $X^1$, $X^2$ und $X^3$ die oben angegebene Bedeutung haben,

mit Salzen von Isothioharnstoffen der Formel (IV)

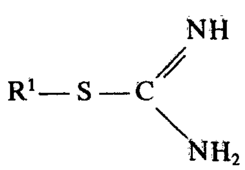

x Z (IV)

in welcher

$R^1$ die oben angegebene Bedeutung hat und
Z für das Äquivalent einer anorganischen Säure steht,

in Gegenwart eines Verdünnungsmittels und in Gegenwart einer Base umsetzt, oder

c) die nach den Verfahren (a) und (b) erhaltenen Triazolylalkyl-thioether-keto-Derivate der Formel (Ia)

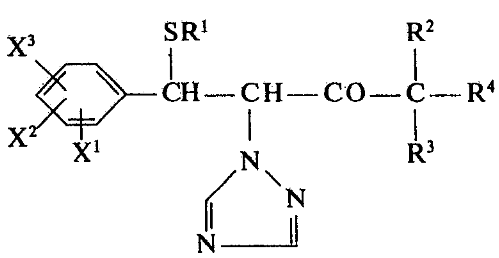

(Ia)

in welcher

$R^1$, $R^2$, $R^3$, $R^4$, $X^1$, $X^2$ und $X^3$ die oben angegebene Bedeutung haben,

im allgemein üblicher Weise reduziert,
und gegebenenfalls anschließend an die Verbindungen der Formel (I) eine Säure oder ein Metallsalz addiert.

3. Pflanzenwuchsregulierende und fungizide Mittel, gekennzeichnet durch einen Gehalt an mindestens einem Triazolylalkyl-thioether der Formel (I) bzw. einem Säureadditionssalz oder Metallsalzkomplex von einem Triazolylalkyl-thioether der Formel (I).

4. Verfahren zur Regulierung des Pflanzenwachstums sowie zur Bekämpfung von Pilzen, dadurch gekennzeichnet, daß man Triazolylalkyl-thioether der Formel (I) bzw. deren Säureadditions-Salze oder Metallsalzkomplexe auf die Pflanzen und/oder deren Lebensraum ausbringt.

5. Verwendung von Triazolylalkyl-thioethern der Formel (I) bzw. von deren Säureadditionssalzen oder Metallsalzkomplexen zur Regulierung des Pflanzenwachstums bzw. zur Bekämpfung von Pilzen.

6. Verfahren zur Herstellung von pflanzenwuchsregulierenden und fungiziden Mitteln, dadurch gekennzeichnet, daß man Triazolylalkyl-thioether der Formel (I) bzw. deren Säureadditionssalze oder Metallsalzkomplexe mit Streckmitteln und/oder Oberflächenaktiven Stoffen vermischt.

7. Triazolylalkyl-thioether der Formel

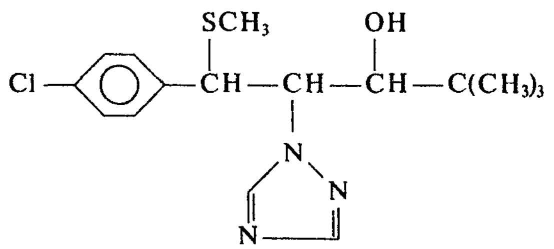

8. Triazolylalkyl-thioether der Formel

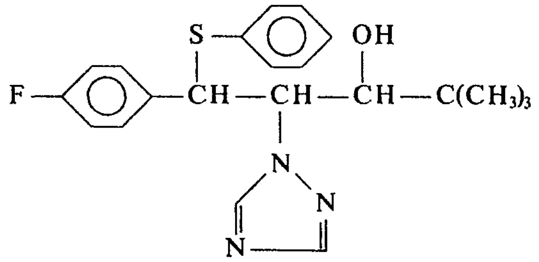

9. Triazolylalkyl-thioether der Formel

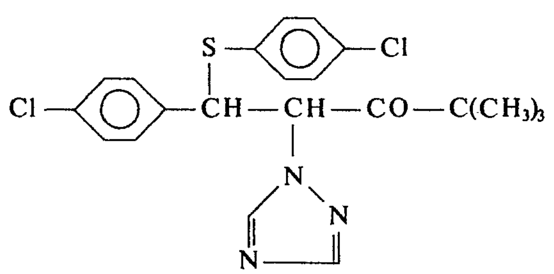

**Claims**

1. Triazolylalkyl-thioethers of the formula (I)

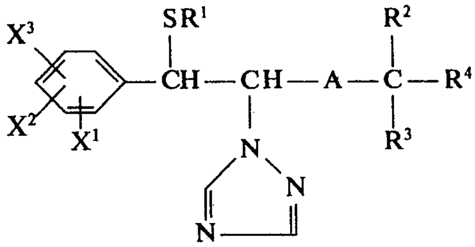

(I)

in which

A represents the keto group or the CH(OH) grouping,
$R^1$ represents alkyl with 1 to 4 carbon atoms or phenyl which is optionally substituted by fluorine, chlorine, bromine and/or alkyl with 1 to 4 carbon atoms,
$R^2$ represents alkyl with 1 to 4 carbon atoms,
$R^3$ represents alkyl with 1 to 4 carbon atoms,
$R^4$ represents alkyl with 1 to 4 carbon atoms, halogenoalkyl with 1 to 4 carbon atoms and 1 to 5 identical or different halogen atoms or alkylthiomethyl with 1 to 4 carbon atoms in the alkyl part,
$X^1$ represents hydrogen, fluorine, chlorine, or bromine,
$X^2$ represents hydrogen, fluorine or chlorine and
$X^3$ represents hydrogen or chlorine,

and their acid addition salts and metal salt complexes.

2. Process for the preparation of triazolylalkyl-thioethers of the formula (I)

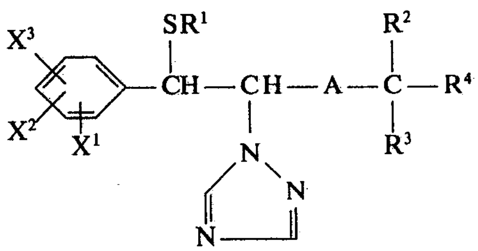

(I)

in which

A represents the keto group or the CH(OH) grouping,
$R^1$ represents alkyl with 1 to 4 carbon atoms or phenyl which is optionally substituted by fluorine, chlorine, bromine and/or alkyl with 1 to 4 carbon atoms,
$R^2$ represents alkyl with 1 to 4 carbon atoms,
$R^3$ represents alkyl with 1 to 4 carbon atoms,
$R^4$ represents alkyl with 1 to 4 carbon atoms, halogenoalkyl with 1 to 4 carbon atoms and 1 to 5 identical or different halogen atoms or alkylthiomethyl with 1 to 4 carbon atoms in the alkyl part,
$X^1$ represents hydrogen, fluorine, chlorine, or bromine,
$X^2$ represents hydrogen, fluorine, or chlorine and
$X^3$ represents hydrogen or chlorine,

and of their acid addition salts and metal salt complexes, characterised in that

a) vinyltriazolyl derivatives of the formula (II)

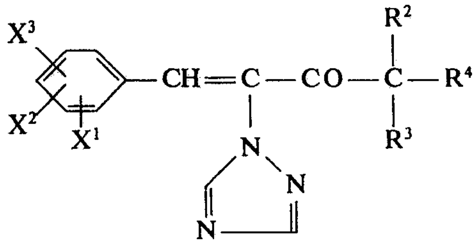

(II)

in which

$R^2$, $R^3$, $R^4$, $X^1$, $X^2$, and $X^3$ have the abovementioned meaning,

are reacted with mercaptans of the formula (III)

$$R^1-S-H \qquad (III)$$

in which

$R^1$ has the abovementioned meaning,

in the presence of a diluent and, if appropriate, in the presence of a base, or

b) vinyltriazolyl derivatives of the formula (II)

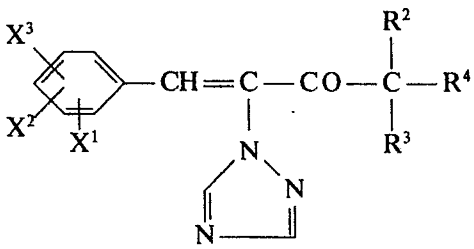

(II)

in which

$R^2$, $R^3$, $R^4$, $X^1$, $X^2$ and $X^3$ have the abovementioned meaning,

are reacted with salts of isothioureas of the formula (IV)

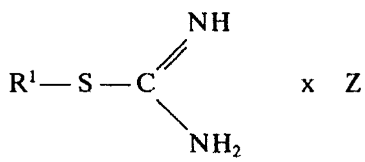

(IV)

in which

$R^1$ has the abovementioned meaning and

Z represents the equivalent of an inorganic acid,

in the presence of a diluent and in the presence of a base, or

c) the triazolylalkyl-thioether-keto derivatives obtained by methods (a) and (b), of the formula (Ia)

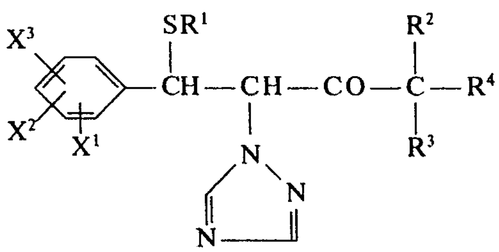

(Ia)

in which

$R^1$, $R^2$, $R^3$, $R^4$, $X^1$, $X^2$ and $X^3$ have the abovementioned meaning,

are reduced in a generally customary manner,
and, if desired, an acid or a metal salt is then added on to the compounds of the formula (I).

3. Plant-growth-regulating and fungicidal agents, characterized in that they contain at least one triazolylalkyl-thioether of the formula (I) or at least one acid addition salt or metal salt complex of a triazolylalkyl-thioether of the formula (I).

4. Method of regulating plant growth and combating fungi, characterized in that triazolylalkyl-thioethers of the formula (I) or their acid addition salts or metal salt complexes are applied to the plants

and/or their habitat.

5. Use of triazolylalkyl-thioethers of the formula (I) or of their acid addition salts or metal salt complexes for regulating plant growth or combating fungi.

6. Process for the preparation of plant-growth-regulating and fungicidal agents, characterized in that triazolylalkyl-thioethers of the formula (I) or their acid addition salts or metal salt complexes are mixed with extenders and/or surface-active substances.

7. Triazolylalkyl-thioethers of the formula

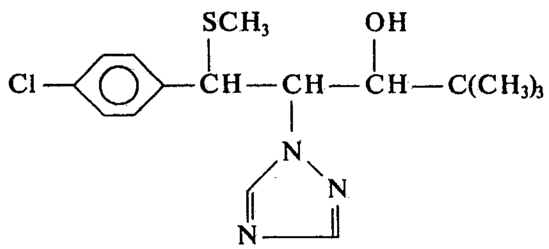

8. Triazolylalkyl-thioethers of the formula

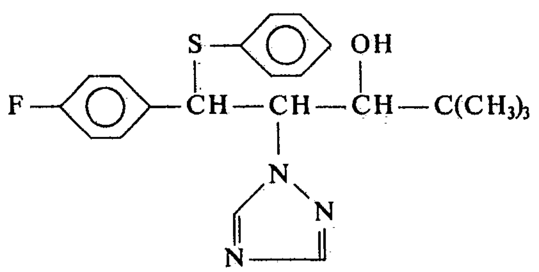

9. Triazolylalkyl-thioethers of the formula

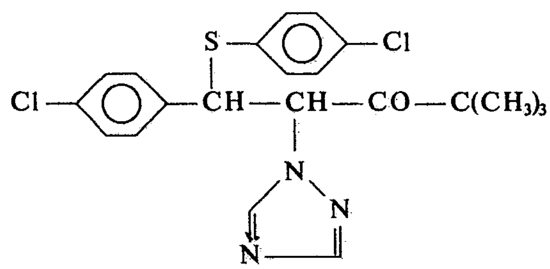

**Revendications**

1. Triazolylalkyl-thioéthers de formule (I)

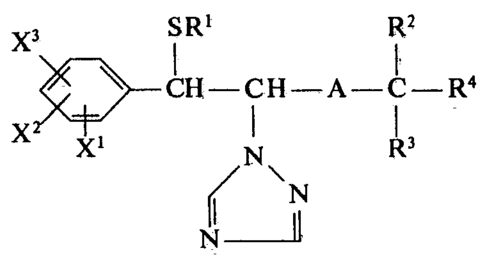
(I)

dans laquelle

A représente le groupe cétonique ou le groupement CH(OH);
$R^1$ représente un groupe alkyle ayant 1 à 4 atomes de carbone ou un groupe phényle (éventuellement substitué par du fluor, du chlore, du brome et/ou un groupe alkyle ayant 1 à 4 atomes de carbone),
$R^2$ représente un groupe alkyle ayant 1 à 4 atomes de carbone,
$R^3$ représente un groupe alkyle ayant 1 à 4 atomes de carbone,
$R^4$ représente un groupe alkyle ayant 1 à 4 atomes de carbone, halogénoalkyle ayant 1 à 4 atomes de

carbone et 1 à 5 atomes d'halogène, identiques ou différents, ou alkylthiométhyle ayant 1 à 4 atomes de carbone dans la partie alkyle,

$X^1$ représente un atome d'hydrogène, de fluor, de chlore ou de brome,

$X^2$ représente un atome d'hydrogène, de fluor ou de chlore, et

$X^3$ représente un atome d'hydrogène ou de chlore,

ainsi que leurs sels d'addition d'acides et complexes avec des sels métalliques.

2. Procédé de préparation de triazolylalkyl-thioéthers de formule (I)

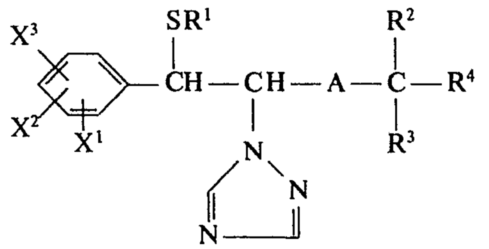

(I)

dans laquelle

A représente le groupe cétonique ou le groupement CH(OH),

$R^1$ représente un groupe alkyle ayant 1 à 4 atomes de carbone ou un groupe phényle (éventuellement substitué par du fluor, du chlore, du brome et/ou un groupe alkyle ayant 1 à 4 atomes de carbone),

$R^2$ représente un groupe alkyle ayant 1 à 4 atomes de carbone,

$R^3$ représente un groupe alkyle ayant 1 à 4 atomes de carbone,

$R^4$ représente un groupe alkyle ayant 1 à 4 atomes de carbone, halogénoalkyle ayant 1 à 4 atomes de carbone et comportant 1 à 5 atomes d'halogène identiques ou différents, ou alkylthiométhyle ayant 1 à 4 atomes de carbone dans la partie alkyle,

$X^1$ représente un atome d'hydrogène, de fluor, de chlore ou de brome,

$X^2$ représente un atome d'hydrogène, de fluor ou de chlore, et

$X^3$ représente un atome d'hydrogène ou de chlore,

ainsi que de leurs sels d'addition d'acides et de leurs complexes avec des sels de métaux, procédé caractérisé en ce que:

a) on fait réagir en présence d'un diluant et éventuellement en présence d'une base des dérivés de vinyltriazolyle de formule (II)

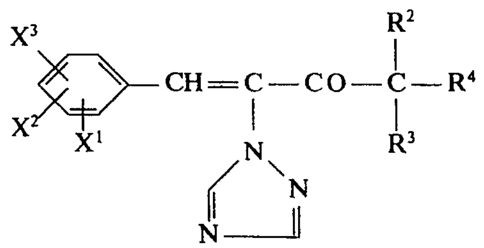

(II)

dans laquelle

$R^2$, $R^3$, $R^4$, $X^1$, $X^2$ et $X^3$ ont le sens indiqué ci-dessus,

avec des mercaptans de formule (III)

$R^1-S-H$ (III)

dans laquelle

$R^1$ a le sens indiqué ci-dessus, ou

b) on fait réagir en présence d'un diluant et en présence d'une base des dérivés de vinyltriazolyle de formule (II)

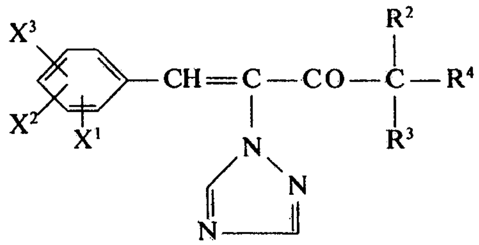

(II)

dans laquelle

$R^2$, $R^3$, $R^4$, $X^1$, $X^2$ et $X^3$ ont le sens indiqué ci-dessus,

avec des sels d'isothiourées de formule (IV)

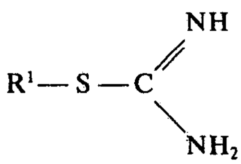

x Z (IV)

dans laquelle

$R^1$ a le sens indiqué ci-dessus, et
Z représente l'équivalent d'un acide minéral, ou

c) on réduit de façon en général usuelle les dérivés cétoniques de triazolylalkyl-thioéthers obtenus selon les procédés (a) et (b), de formule (Ia)

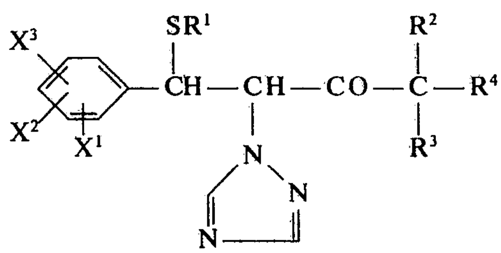

(Ia)

dans laquelle

$R^1$, $R^2$, $R^3$, $R^4$, $X^1$, $X^2$ et $X^3$ ont le sens indiqué ci-dessus,

et éventuellement, on fixe ensuite par addition sur les composés de formule (I) un acide ou un sel de métal.

3. Produit à rôle de substance de croissance et à action fongicide, caractérisé par une teneur en au moins un triazolylalkyl-thioéther de formule (I) ou en un sel d'addition d'acide ou un complexe avec un sel de métal de triazolylalkyl-thioéther de formule (I).

4. Procédé de régulation de la croissance des plantes et pour lutter contre les champignons, caractérisé en ce qu'on applique des triazolylalkyl-thioéthers de formule (I) ou leurs sels d'addition d'acides ou leurs complexes avec des sels de métaux sur les plantes et/ou sur leur biotope ou espace vital.

5. Utilisation des triazolylalkyl-thioéthers de formule (I) ou de leurs sels d'addition d'acides ou de leurs complexes avec des sels de métaux pour la régulation de la croissance des plantes ou pour combattre des champignons.

6. Procédé de préparation de produits à rôle de substance de croissance et à action fongicide, procédé caractérisé en ce qu'on mélange des triazolylalkyl-thioéthers de formule (I) ou leurs sels d'addition d'acides ou leurs complexes avec des sels de métaux avec des agents d'allongement et/ou des substances tensioactives.

7. Triazolylalkyl-thioéther de formule:

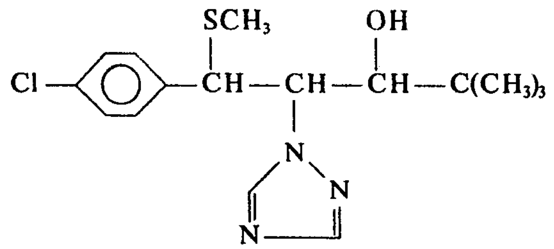

8. Triazolylalkyl-thioéther de formule:

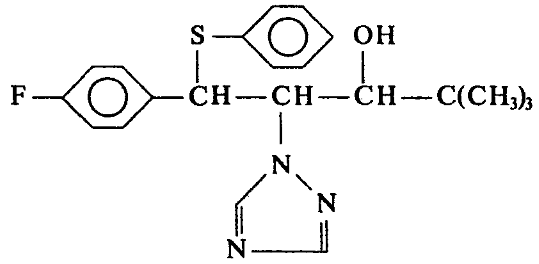

9. Triazolylalkyl-thioéther de formule: